## Europäisches Patentamt
## European Patent Office
## Office européen des brevets

(19)

(11) Numéro de publication: **0 028 172**
**B1**

(12) **FASCICULE DE BREVET EUROPEEN**

(45) Date de publication du fascicule du brevet: 07.09.83

(51) Int. Cl.³: **A 61 K 39/108**

(21) Numéro de dépôt: 80401402.5

(22) Date de dépôt: 03.10.80

(54) Vaccin animal anticolibacillaire, obtention, application et souches d'escherichia coli utilisées.

(30) Priorité: 03.10.79 FR 7924665

(43) Date de publication de la demande: 06.05.81 Bulletin 81/18

(45) Mention de la délivrance du brevet: 07.09.83 Bulletin 83/36

(84) Etats contractants désignés: BE DE FR GB IT NL

(56) Documents cités:
EP-A-0 003 692
FR-A-2 119 903
FR-A-2 208 648
FR-A-2 282 908
FR-A-2 347 932
US-A-3 907 987
CHEMICAL ABSTRACTS, vol. 67, 18-12-1967 page 10850, réf.: 115267t, no. 25 Columbus, Ohio, US A. KOSTAKEV: »Vaccination of pregnant cows and its effect on the intestinal microflora in new-born calves with dyspepsia«
CHEMICAL ABSTRACTS, vol. 76, no. 13, 27-03-1972, page 261, réf.: 70908v Columbus, Ohio, US C.M. CAMERON et al.: »Immune response to

(73) Titulaire: Etablissement public dit: INSTITUT NATIONAL DE LA RECHERCHE AGRONOMIQUE, 149, rue de Grenelle, F-75341 Paris Cedex 07 (FR)

(72) Inventeur: **Gouet, Philippe, Laschamps-St-Genès Champanelle, F-63110 Beaumont (FR)**
Inventeur: **Contrepois, Michel, Gilbert, 55, Hameau de Laniraud Blanzat, F-63100 Clermont-Ferrand (FR)**
Inventeur: **Dubourguier, Henri-Charles, 22, rue des 3 Résistants, F-63100 Clermont-Ferrand (FR)**
Inventeur: **Girardeau, Jean-Pierre, 8 Avenue Jean Moulin, F-63540 Romagnat (FR)**
Inventeur: **Goby, Jean-François, décédé (FR)**

(74) Mandataire: **Phélip, Bruno et al, c/o Cabinet Harlé & Phélip 21, rue de La Rochefoucauld, F-75009 Paris (FR)**

dead and live Escherichia coli vaccines and colostral transfer of immunity to calves and lambs«
UNLISTED DRUGS, vol. 21, no. 12, décembre 1969, page 189, réf. 2, »Porcovac« Vet Rec 85: II, 6 sep. 69 (advt.)
CHEMICAL ABSTRACTS, vol. 87, no. 5, 01-08-1977, page 19, réf.: 33523x Columbus, Ohio, US J. KRAJC: »Evaluation of some therapeutic and prophylactic measures for diarrhea control in calves«

BUNDESDRUCKEREI BERLIN

## Vaccin animal anticolibacillaire, obtention,
## application et souches d'escherichia coli utilisées

La présente invention concerne le domaine des vaccins animaux. Elle concerne plus particulièrement un vaccin permettant la prophylaxie anticolibacillaire des diarrhées des animaux nouveau-nés, tels que les veaux.

L'invention a également pour objet un procédé d'obtention d'un tel vaccin, des préparations vaccinales formulées en vue de l'administration ainsi que les applications desdites préparations.

De nombreux travaux ont été effectués depuis 1960 et surtout depuis 1970, pour étudier le rôle des E.coli entéropathogènes sur les diarrhées des animaux nouveau-nés, et on a déjà proposé ou commercialisé, des vaccins correspondant aux divers types d'antigènes isolés dans ces bactéries. Les références bibliographiques illustrant l'art antérieur sont nombreuses. L'homme de l'art pourra par exemple se reporter à l'article de S.D. ACRES et al »Immunization of Calves Against Enterotoxigenic Colibacillosis by Vaccinating Dams with Purified K99 Antigen and Whole Cell Bacterins« dans »Infection and Immunity« july 1979 — page 121 – 126 Vol. 25 n° 1. Cet article cite un nombre important de publications antérieures pertinentes, qui sont également introduites à titre de référence dans la présente description. A titre complémentaire, et sans que la liste ci-après présente un caractère exhaustif, on peut encore citer les références suivantes:

J.M. RUTTER (1975)
Escherichia coli infections in piglets: Pathogenesis, virulence and vaccination
The Vet. Record. 22 february 1975
M.R. WILSON (1974)
Immunity to E. coli in pigs; efficiency of a live formalized vaccine under field conditions
Brit. vet. J 130 page 599
J.L. NAGY, P.D. WALKER, B.S. BHOGAL, T. MacKENZIE
Evaluation of E. coli vaccines against experimental enteric colibacillosis
Wellcome — Europe informations — n° 6 automne 1978
Un vaccin qui empêche l'adhésion des bactéries
I. ORSKOV, F. ORSKOV, H.W. SMITH, W.J. SOJKA (1975)
The establishment of K99, a thermolabile transmissible E. coli K antigen, previously called »Kco« possessed by calf and lambs enteropathogenic strains.
Acta.part. microbiol. scand. Sect. B 83 31 – 36 (1975) P à M Guinee — J. VELDKAMP — W.H. JANSEN (1977)
Improved Minca medium for the detection of K99 antigen in calf enterotoxigenic strains of E. coli
Inf. & Immunity 15,2 676 – 678
J.A. MORRIS, A.E. STEVENS, W.J. SOJKA (1977)
Preliminary characterization of cell free K99 antigen isolated from E. coli B 41
J. of Gen. Microbiol. 99 353 – 357
L.L. MYERS (1976)
Vaccination of cows with an E.coli bacterin for the prevention of naturally occuring diarrheal disease in their calves
Am. J. of Vet. Res 37, 7 831, 834
W.J. SOJKA, C. WRAY, J.A. MORRIS (1978)
Passive protection of lambs against experimental enteric colibacillosis by colostral transfer of antibodies from K99 vaccinated ewes
J. Med. Microbiol. 11 493, 499
J.W. COSTERTON, G.G. GEESEY, K.J. CHENG (1978) Comment collent les bactéries
Pour la science n° 5

En particulier, le dernier article mentionné, extrait de la revue »Pour la Science« n° 5 de 1978, illustre les propriétés d'attachement des bactéries aux épithéliums.

On présentera maintenant un bref résumé des connaissances acquises dans l'art antérieur dans le domaine des vaccinations anticolibacillaires. La bactérie porte des flagelles présentant l'antigène H. Elle peut présenter également des antigènes protéiques particuliers du type, par exemple, K88 ou K99. L'enveloppe capsulaire porte des lantigènes K. Quant à la paroi bactérienne, elle porte les antigènes O ou somatiques. Les travaux de ORSKOV, au Centre International des entérobactéries du Danemark, ont permis de mettre en évidence un antigène particulier K88 dont la synthèse est codée par un plasmide. Cet antigène confère à E.coli des propriétés d'attachement à l'épithélium intestinal du porcelet. A la suite de ces travaux, on a proposé et mis sur le marché, des vaccins applicables aux truies et contenant l'antigène K88. Ces vaccins stimulent la protection conférée par les anticorps colostraux. On a mis en évidence de la même manière, d'autres structures d'E. coli appelées 987 P responsables de l'attachement de E. coli à l'épithélium intestinal du porcelet.

On a mis également en évidence, un antigène K commun (Kco) aux souches d'E. coli entéropathogènes d'origine bovine ou ovine. Cet antigène a été caractérisé et inclus dans la nomenclature internationale sous l'appellation de K99. Sa synthèse est également codée par un plasmide. (Voir en particulier la publication de I. ORSKOV et al »the establishment of K99, a thermolabile, transmissible E.coli K antigen, previously called »Kco« possessed by calf and lambs enteropathogenic strains« Acta. path. microbiol. scand. Sect. B 83 31—36 1975.) On a ensuite proposé des milieux de culture spécialement appropriés pour la recherche des E.coli K99+. On a également réalisé la caractérisation biochimique de l'antigène K99. Il ressort des publications citées ci-dessus ainsi que d'autres, que l'antigène K99 est maintenant défini et accessible d'une manière parfaitement suffisante à l'homme de l'art.

Dans le domaine des applications pratiques, en particulier en vue de la prophylaxie anticolibacillaire chez les bovins, on a proposé et commercialisé de nombreux vaccins correspondant aux divers typés sérologiques O (antigène de la paroi) et parfois de la capsule (antigène K). On a par exemple réalisé une vaccination à l'aide de la souche d'E.coli B 44 dont on a montré qu'elle contenait l'antigène K99. Les résultats d'une telle vaccination sont satisfaisants pour un certain nombre de troupeaux, mais ne permettent pas une prophylaxie complète des animaux traités. Les principales études ont porté sur la protection des veaux, mais on a également utilisé des vaccins à base d'antigène K99 pour la protection colostrale d'agneaux par vaccination de brebis.

D'une manière générale, on a jusqu'à présent fait appel aux antigènes K88, K99, 987 P et pratiquement à tous les »pili« d'E.coli. Il est maintenant connu que le facteur d'attachement aux épithéliums joue un rôle très important dans le développement des prophylaxies. Jusqu'à présent, cependant, il est difficile de mettre expérimentalement en évidence les propriétés d'attachement d'E.coli. Dans le cadre de ses travaux concernant la purification et la structure des antigènes d'attachement K88 et K99, J.P. GIRARDEAU a mis au point un modèle in vitro sur villosités intestinales isolées faciles à préparer qui permettent de visualiser au microscope, après contact avec une suspension d'E.coli l'attachement ou non sur la villosité. Ce modèle in vitro a permis de montrer que les bactéries possédant l'antigène K99 s'attachent, mais que d'autres bactéries K99 — possèdent aussi cette propriété. La technique de GIRARDEAU, décrite dans Ann. Microbiol. Institut Pasteur, 1980, 1318, 31—37, consiste à utiliser des villosités isolées d'intestins animaux (lapins ou veaux) conservées par congélation ou dans un tampon formolé. Ces villosités, mises en contact 15 minutes avec les bactéries à étudier, sont ensuite observées en microscopie optique à contraste de phase. On peut distinguer facilement la bordure en brosse de la villosité et les bactéries qui s'y sont attachées. L'intensité de l'attachement est appréciée par comptage des bactéries fixées au bord de la villosité.

La présente invention, mettant à profit la technique proposée par GIRARDEAU pour mettre en évidence de nouveaux systèmes d'attachement d'E.coli, a tout d'abord permis d'isoler et de caractériser des souches sauvages d'E.coli sur des veaux atteints de diarrhée. Lesdites souches sont appelées Y+, 31A+ et 47A+. Des souches-types sont respectivement inscrites dans la Collection Nationale de Cultures de Microorganismes (CNCM) détenue à l'Institut Pasteur-28, rue du Docteur Roux-Paris 15ème, où elles ont reçu un numéro d'enregistrement au catalogue, respectivement I-104, I-105 et I-106.

Les souches nouvelles mises en oeuvre selon l'invention ont été caractérisées, ainsi qu'il sera montré ci-après, par leur aptitude à fournir des antigènes spécifiques. Les autres caractéristiques des souches, connues du demandeur, sont indiquées dans les tableaux ci-après.

TABLEAU I

| Sou-che | Antigènes | | | | Entéro-toxine Tsa*) | Profil API**) | Antibiogramme***) | | | | | | | |
|---|---|---|---|---|---|---|---|---|---|---|---|---|---|---|
| | K99 | Y | 31A | 47A | | | A | S | K | C | T | Su | Tnp | Gen |
| 11A (Y+) | + | + | − | − | + | 5144552.1 | − | + | + | + | + | + | − | − |
| 31A | − | − | + | − | − | 5144572.1 | − | + | + | + | + | + | − | − |
| 47A | − | − | − | + | − | 5144552.1 | + | + | − | − | + | + | − | − |

*) Entérotoxine thermostable a
**) Profil API établi sur 24 caractères
***) Antibiogramme − = sensible
                    + = résistant

A   = ampicilline
S   = streptomycine
K   = Kanamycine
C   = chloramphénicol
T   = tétracycline
Su  = sulfamide
Tnp = triméthoprime
Gen = gentamycine

TABLEAU II
Caractéristiques biochimiques des souches types

| | 11A | 31A | 47A |
|---|---|---|---|
| Glycérol | + | + | + |
| Erythritol | − | − | − |
| D-arab | ± | + | + |
| L-arab | + | + | + |
| Ribose | + | + | + |
| D-xylose | + | + | + |
| L-xylose | − | − | − |
| Adonitol | + | − | − |
| Méthyl-xyloside | − | − | − |
| galactose | + | + | + |
| D-glucose | + | + | + |
| levulose | + | + | + |
| D-mannose | + | + | + |
| L-sorbose | − | + | + |
| Rhamnose | + | + | + |
| Dulcitol | − | + | + |

4

Fortsetzung

| | 11A | 31A | 47A |
|---|---|---|---|
| Méso inositol | — | + | — |
| Mannitol | + | + | + |
| Sorbitol | + | + | + |
| Méthyl-D mannoside | — | — | — |
| Méthyl-D glucoside | — | — | — |
| N-acétyl-glucosamide | + | + | + |
| Anygdaline | — | — | — |
| Arbutine | ± | — | — |
| Esculine | + | — | — |
| Salycone | + | — | — |
| D-cellubiose | — | — | — |
| Maltose | + | + | + |
| Lactose | + | + | + |
| D-mélibiose | + | + | + |
| Saccharose | — | + | + |
| D-tréhalose | + | + | + |
| Inuline | — | — | — |
| D-mélezitose | — | — | — |
| D-raffinose | — | + | + |
| Dextrine | ± | — | — |
| Amylose | — | — | — |
| Amidon | — | — | — |
| Glycogène | — | — | — |

TABLEAU III

caractéristiques biochimiques de souches sauvages (polulations)

| | Sérologie négative*) | K99 | K99-Y | 31A-Y | 31A | Y |
|---|---|---|---|---|---|---|
| Nombre de souches | 169 | 11 | 17 | 23 | 54 | 37 |
| Saccharose | 56,2 | 0 | 5,9 | 56,5 | 57,4 | 75,7 |
| Raffinose | 65,7 | 0 | 5,9 | 91,3 | 94,4 | 78,4 |
| Dulcitol | 50,9 | 0 | 5,9 | 47,8 | 88,9 | 40,5 |
| Inositol | 28,4 | 0 | 0 | 95,6 | 59,2 | 43,2 |
| Sorbitol | 68,6 | 27,3 | 5,9 | 65,2 | 64,8 | 70,3 |
| Ampicilline | 66,3 | 90,9 | 100 | 69,6 | 63 | 86,5 |
| Carbénicilline | 53,0 | 72,3 | 17,6 | 56,5 | 61,1 | 70,3 |
| Céphalexine | 20,7 | 27,3 | 82,4 | 60,9 | 1,8 | 59,4 |
| Streptomycine | 83,4 | 100 | 82,4 | 100 | 90,4 | 94,6 |
| Kanamycine | 65,1 | 72,7 | 82,4 | 91,3 | 59,3 | 89,2 |
| Chloramphénicol | 66,3 | 100 | 82,4 | 91,3 | 88,8 | 89,2 |
| Tétracycline | 75,7 | 100 | 35,3 | 100 | 96,3 | 86,5 |
| Sulfamides | 84,6 | 100 | 82,4 | 100 | 100 | 100 |
| Nalidixique | 0 | 0 | 0 | 0 | 1,8 | 0 |
| Triméthoprime | 18,9 | 0 | 0 | 60,9 | 5,5 | 43,2 |
| Colicine | 35,5 | 36,4 | 0 | 56,5 | 31,5 | 37,8 |

*) Sérologie négative signifie qu'on n'a pas identifié un antigène d'attachement.

Les chiffres du tableau III indiquent le pourcentage de souches présentant le caractère considéré.

Certaines souches portent les deux antigènes K99 et Y. Il est donc important de disposer d'un vaccin fournissant des anticorps contre ces deux antigènes.

Les tableaux II et III ci-dessus fournissent respectivement les caractéristiques des souches-types et des souches sauvages. Il va sans dire qu'on peut utiliser selon l'invention une souche sauvage, au lieu de la souche-type correspondante, dès lors qu'elle présente des caractéristiques générales similaires à cette dernière.

La présente invention a pour objet un vaccin anticolibacillaire ayant une efficacité accrue par rapport aux vaccins antérieurs, lesquels n'étaient pas capables d'immuniser les animaux traités contre les structures d'attachement d'E. coli essentiellement responsables des désordres intestinaux chez ces animaux.

L'invention concerne donc un vaccin anticolibacillaire animal caractérisé en ce qu'il contient l'antigène K99 et au moins un antigène provenant des souches d'E.coli choisies parmi les souches Y+, 31A+ et 47A+.

Ainsi, un vaccin hautement préféré selon l'invention, contient les antigènes K99 et Y en association ou non avec les antigènes 31A et/ou 47A. En variante, le vaccin peut contenir les antigènes K99 et 31A, en association ou non avec les antigènes Y et/ou 47A.

L'invention a également pour objet les nouvelles souches d'E.coli portant les antigènes 31A, Y et 47A.

**0 028 172**

Une souche d'E.coli conforme à l'invention est caractérisée en ce qu'elle comporte l'antigène de surface F (31A) reconnaissable en séroagglutination à l'aide d'un antisérum spécifique de cet antigène, préparé à partir de la souche type (n° de collection I-105 à l'Institut Pasteur), ledit antisérum ne reconnaissant ni l'antigène K99 (souche de référence B41) ni l'antigène Y (n° collection I-104) ni l'antigène 47A (n° collection I-106).

Une autre souche d'E.coli conforme à l'invention est caractérisée en ce qu'elle comporte l'antigène de surface F (Y) reconnaissable en séroagglutination à l'aide d'un antisérum spécifique de cet antigène, préparé à partir de la souche type (n° de collection I-104), ledit antisérum ne reconnaissant ni l'antigène K99 (souche de référence B41) ni l'antigène 31A (n° de collection I-105) ni l'antigène 47A (n° de collection I-106).

Une autre souche selon l'invention est caractérisée en ce qu'elle comporte l'antigène de surface F(47A) reconnaissable en séroagglutination à l'aide d'un antisérum spécifique de cet antigène, préparé à partir de la souche type (n° de collection I-106), ledit antisérum ne reconnaissant ni l'antigène K99 (souche de référence B41) ni l'antigène 31A (n° de collection I-105) ni l'antigène Y (n° de collection I-104).

Les antigènes de surface portés par les souches de E. coli utilisées selon l'invention sont observables en microscopie électronique. Cette méthode constitue donc un des moyens préliminaires pour reconnaître l'existence de telles structures d'attachement sur les E.coli.

Une technique très intéressante et originale d'étude de l'attachement in vitro consiste à utiliser des villosités isolées de jeunes animaux (lapins ou veaux) conservées par congélation, ou dans un tampon formolé. Ces villosités mises en contact 15 mn avec les bactéries à étudier sont ensuite observées en microscopie optique à contraste de phase. On peut distinguer facilement la bordure en brosse de la villosité et les bactéries qui s'y attachées. L'intensité de l'attachement est appréciée par comptage des bactéries fixées sur le bord de la villosité. La compétition entre les sites récepteurs et les substances additionnées dans le tampon peut entraîner l'inhibition de l'attachement et mettre ainsi en évidence les molécules impliquées dans l'attachement. Ainsi a-t-on pu mettre en évidence l'inhibition de l'attachement de souches Y+ par la N-acétyl-glucosamine sur des villosités de veau.

L'utilisation de compétiteurs ayant plus d'affinité pour le site d'attachement rend la localisation du récepteur possible. La lectine de germe de blé, spécifique de la N-acétyl-glucosamine, inhibe l'attachement Y à partir de 100 μg. Cette inhibition est due au site récepteur localisé sur la villosité intestinale et l'antigène d'attachement reconnaît ce site par une structure stéréospécique de nature protéique.

Les antigènes Y, 31A et 47A, présentent plus d'affinité pour les villosités de veaux, alors que l'antigène K99 présente moins de spécificité entre villosités d'origines différentes. En microscopie électronique, l'antigène Y se rapproche de l'antigène K99, bien que plus dense, avec formation de gros paquets de fibres autoagglutinées. Les autres antigènes 31A et 47A ressemblent plus à des »pili«, bien que leurs dimensions soient nettement plus faibles. En général, les souches portant ces nouvelles structures ne sont pas entérotoxynogènes (dans le modèle du souriceau) mais leur fréquence chez des animaux ayant des troubles digestifs est importante. A titre illustratif, un sondage réalisé sur un troupeau expérimental, a montré que sur 20 veaux atteints de diarrhée, 13 étaient porteurs d'une souche d'E.coli Y+.

En vue de la protection des animaux nouveau-nés, le vaccin selon l'invention est pratiqué sur la mère. L'animal nouveau-né trouve alors protégé lorsqu'il absorbe le colostrum de sa mère.

L'invention a également pour objet un procédé pour l'obtention du vaccin défini précédemment. Conformément à ce procédé, on cultive séparément les souches d'E.coli portant les antigènes considérés, on récolte les bactéries ainsi respectivement cultivées, on mélange les cultures de bactéries respectives, on inactive le mélange de bactéries et on l'additionne éventuellement d'un adjuvant, ce qui conduit à la préparation vaccinale désirée.

Dans la pratique, la souche d'E.coli portant l'antigène K99 peut avantageusement être la souche B41, connue de l'homme de l'art, et mise en évidence au Centre International des Entérobactéries à Copenhague. Les autres souches portent les antigènes Y, 31 A et 47 A, sont originales et ont été définies précédemment.

Au cours des travaux qui ont donné naissance à la présente invention, on a également trouvé un milieu de culture spécialement approprié aux souches d'E.coli mises en oeuvre, en particulier de la souche portant l'antigène K99. On a découvert en effet, que la synthèse de l'antigène K99 est inhibée en présence de L-alanine. Un milieu de culture préféré est donc un milieu aqueux gélosé, tamponné, contenant entre autres des acides aminés, du glucose et des éléments minéraux, ledit milieu étant plus particulièrement remarquable en ce qu'il contient au plus 0,3 g/litre d'alanine. Le milieu contient avantageusment, à titre complémentaire, de l'acide aspartique.

Sous une forme préférée de réalisation, le milieu de culture présente la composition suivante en g/litre:

| | |
|---|---|
| — milieu gélosé | 10 à 20 g d'agar |
| — milieu tamponné | à pH 7,5 (phosphates) |
| — acides aminés (peptone) | 1 à 5 g |

7

| | |
|---|---|
| — glucose | 1 à 5 g |
| — acide aspartique | 0 à 4 g |
| — Désoxycholate de Na | 0 à 2 g |
| — Extraits de levures | 0 à 1 g |
| — Eléments minéraux (MgSO$_4$, MnCl$_2$, FeCl$_2$, CaCl$_2$) | de 0,01 à 0,0001 g |

Si désiré, le milieu de culture considéré peut être autoclave à 120°C.

La culture des bactéries E.coli est effectuée à la surface de la gélose, avec ou sans film aqueux (1 mm d'épaisseur).

Après incubation, à une température voisine de 37°C, les bactéries sont récoltées à la surface de la gélose, et amenées par exemple à une concentration de $10^9$ à $10^{10}$/ml.

Les cultures se font de la manière indiquée ci-dessus séparément pour chaque souche contenant l'antigène utilisé dans le vaccin selon l'invention. Après récolte des bactéries respectives, celles-ci sont mélangées pour fournir la préparation vaccinale contenant la combinaison d'antigènes prévue selon l'invention.

L'étape consécutive du procédé consiste à inactiver les bactéries E.coli. A cet effet on utilise les moyens d'inactivation connus dans le domaine des vaccins, c'est-à-dire soit des moyens chimiques, soit des moyens physiques. En ce qui concerne les moyens chimiques, on utilise avantageusement le formaldéhyde. De préférence, on utilise la solution commerciale (à 30% des formaldéhyde), à raison de 0,5% de ladite solution par rapport au volume total du milieu à traiter, auquel cas la durée d'inactivation à 37°C est d'environ 24 heures. Les moyens physiques peuvent consister en une irradiation par des rayons gamma. La dose de rayonnement dépendra de la concentration bactérienne de la préparation. Lorsque celle-ci est de l'ordre de $10^9$ à $10^{10}$/ml, la dose de rayons gamma peut être de 0,1 à 1 Mrad. L'irradiation à l'aide de rayons gamma a donné les meilleurs résultats.

Il est souvent avantageux d'ajouter à la préparation vaccinale contenant les bactéries inactivées, un adjuvant du type connu dans cette technique, par exemple de l'hydroxyde d'aluminium. On peut par exemple ajouter de l'hydroxyde d'aluminium colloïdal à raison de 1 à 3 mg/ml par rapport à la préparation liquide finale. Complémentairement, on peut ajouter aussi de la saponine, auquel cas la concentration de saponine sera de 0,5 à 3 mg pour 2 à 5 ml de la préparation totale.

Dans la préparation vaccinale finale, les proportions des bactéries inactivées portant les antigènes respectifs K99, Y, 31A et 47A, ne sont pas critiques. D'ailleurs, certaines bactéries E.coli portant l'antigène K99 peuvent également porter l'antigène Y. En règle générale, cependant, on préfère que les divers antigènes soient présents en proportions sensiblement égales dans la préparation vaccinale.

La préparation vaccinale de l'invention s'utilise en injection sous-cutanée.

L'invention concerne aussi l'application du vaccin et de la préparation vaccinale définies précédemment, en vue de la prophylaxie anticolibacillaire des diarrhées de l'animal nouveau-né, en particulier du veau nouveau-né. Bien que les résultats les plus intéressant aient été obtenus chez le veau, l'invention est également applicable au traitement des porcelets et des agneaux. En vue de son application, la préparation vaccinale est injectée, notamment par voie sous-cutanée, aux mères gestantes, à une période se situant entre 15 et 90 jours, de préférence entre 20 et 40 jous avant la mise bas, dans le cas de la prophylaxie des veaux. Sur le terrain, 2 à 5 ml de la préparation vaccinale contenant 5 × $10^9$ à 5 × $10^{10}$ bactéries au total sont injectés aux mères gestantes.

Les résultats des essais de vaccination effectués sur le terrain montrent l'effet protecteur des colostrums de vaches vaccinées sur des veaux nouveau-nés. On a constaté que la préparation vaccinale était bien tolérée par les mères et permettait d'obtenir des titres élevés d'anticorps dans le colostrum. On n'a pas relevé de chocs anaphylactiques chez les vaches immunisées.

L'invention sera maintenant illustrée sans être aucunement limitée, à l'aide des exemples ci-après. Les exemples 1 à 4 concernent l'obtention respectivement des vaccins de types A, B, C, D, qui ont été utilisés pour des essais de vaccination anticolibacillaire dans un troupeau de vaches allaitantes, les veaux étant infectés à la naissance, dans les conditions rapportées à l'exemple 5.

Exemple 1

Obtention de la préparation vaccinale A

Cet exemple concerne l'obtention d'une préparation vaccinale contenant des bactéries E.coli inactivées par irradiation et l'alumine comme adjuvant.

On a utilisé la souche B41 (connue) portant l'antigène K99 et deux des souches originales selon l'invention, portant respectivement les antigènes Y et 31A. On a d'abord effectué une culture séparée des trois souches pour produire les cellules bactériennes. Dans chaque cas le milieu de culture utilisé avait la composition ci-après (rapportée à un litre)

| | |
|---|---|
| — milieu gélosé | 20 g d'agar |
| — milieu tamponné | à pH 7,5 (phosphates) |

8

| | |
|---|---|
| — acides aminés (peptone) | 1 g |
| — glucose | 1 g |
| — acide aspartique | 0 à 2 g |
| — extraits de levures | 1 g |
| — éléments minéraux (MgSO$_4$, MnCl$_2$, FeCl$_2$, CaCl$_2$) | 0,01 à 0,0001 g |

Les cultures sont séparément effectuées à la surface de la gélose sans film aqueux. Après incubation à 37°C, les bactéries des cultures respectives sont récoltées à la surface de la gélose et amenées à une concentration de 5 · 10$^9$ pour chacune des souches.

Les bactéries récoltées sont ensuite mélangées et inactivées par irradiation gamma, à la dose de 0,6 Mrad. La concentration bactérienne était de 5 · 10$^9$/ml.

On ajoute enfin à la solution finale 2 mg/ml d'alumine colloïdale.

On obtient ainsi, le vaccin A.

### Exemple 2

#### Obtention de la préparation vaccinale B

On opère comme dans l'exemple 1 ci-dessus, sauf que pour obtenir le vaccin final, on ajoute complémentairement de la saponine à titre d'adjuvant, à raison de 2 mg par rapport à la préparation finale ajustée à un volume de 5 ml. On obtient ainsi le vaccin B.

### Exemple 3

#### Obtention de la préparation vaccinale C

On opère comme dans l'exemple 1 ci-dessus, mais au lieu d'inactiver les bactéries par irradiation gamma, on traite le mélange bactérien récolté par 0,5% de formol pendant 24 heures à 37°C. Après inactivation des bactéries, on introduit à titre d'adjuvant de l'alumine colloïdale à raison de 2 mg/ml de la solution finale.

### Exemple 4

#### Obtention de la préparation vaccinale D

On opère comme dans l'exemple 3 ci-dessus, sauf qu'on ajoute complémentairement à la préparation contenant de l'alumine, de la saponine à titre d'adjuvant à raison de 2 mg par rapport à la solution finale (volume total: 5 ml)

### Exemple 5

#### Immunisation des vaches gestantes

Les vaccins, A, B, C, D, préparés respectivement aux exemples 1 à 4 ci-dessus, ont été utilisés pour des expériences sur le terrain. A titre de témoin on a utilisé une préparation contenant seulement un mélange d'alumine et de saponine. En vue de la vaccination on injecte par voie sous-cutanée aux mères gestantes 5 ml de chacune des préparations précédemment définies. La période choisie avant la mise bas se situe entre 15 et 90 jours.

#### Resultats

#### a) Inocuité

Quel que soit le type de préparation, les vaches n'ont présenté aucun symptôme anormal. Une perte d'appétit légère et passagère est apparue quelques heures après l'injection chez certaines d'entre elles.

Dans les lots A et B, aucune anomalie au cours de la gestation ou de la naissance n'a été observée.

Dans le lot D, l'un des veaux a présenté une occlusion intestinale à la naissance.

Dans le lot C, une vache n'avait pas de lait (césarienne) un veau est mort à la naissance et un veau est né anorexique.

Dans le lot »témoin«, une vache a avorté à 8 mois environ et un veau est mort à la naissance.

9

### b) Pouvoir immunisant

Les anticorps ont été recherchés dans le sérum et le colostrum des vaches témoins ou vaccinées. Chez deux vaches témoins ayant mis bas tardivement, on a trouvé des anticorps anti K99 et anti Y à la mise bas dans le sérum et dans le colostrum. Ceci peut être lié soit à une contamination par les E.coli entéropathogènes présents dans l'étable expérimentale, soit à une immunisation naturelle antérieure. Les autres vaches témoins ne présentaient pas d'anticorps sériques ou colostraux.

Quel que soit le type du vaccin, le réponse immunitaire des vaches a été généralement bonne aprés une seule injection. Les titres obtenus étaient compris entre 1/80 et 1/160 pour les anti K99 et 1/160 et 1/320 pour les anti Y.

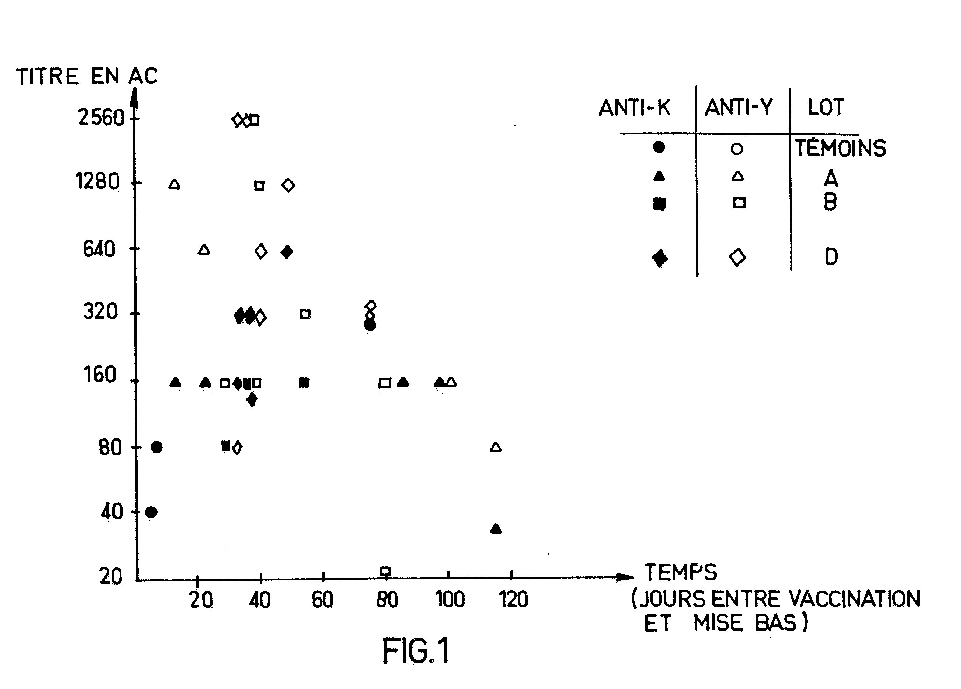

Dans la figure 1, on a reporté les titres obtenus dans le colostrum contre deux antigènes utilisés en fonction du délai entre vaccination et mise bas. La figure 1 est un graphique où l'on a porté en ordonnées le titre d'anticorps Ac dans le colostrum, et en abscisses le nombre de jours séparant la vaccination et la mise bas. Les titres en anticorps colostraux sont généralement supérieurs aux titres sériques (concentration dans la mamelle). L'apparition des anticorps dans le colostrum est très rapide puisque 12 jours suffisent à obtenir des titres appréciables après la vaccination. La période de vaccination idéale se situe entre le 20e et le 40e jour avant la mise bas. Passé ce délai, le titre diminue notablement mais reste cependant important jusqu'au 80e jour après la vaccination.

### c) Persistance des anticorps dans le lait

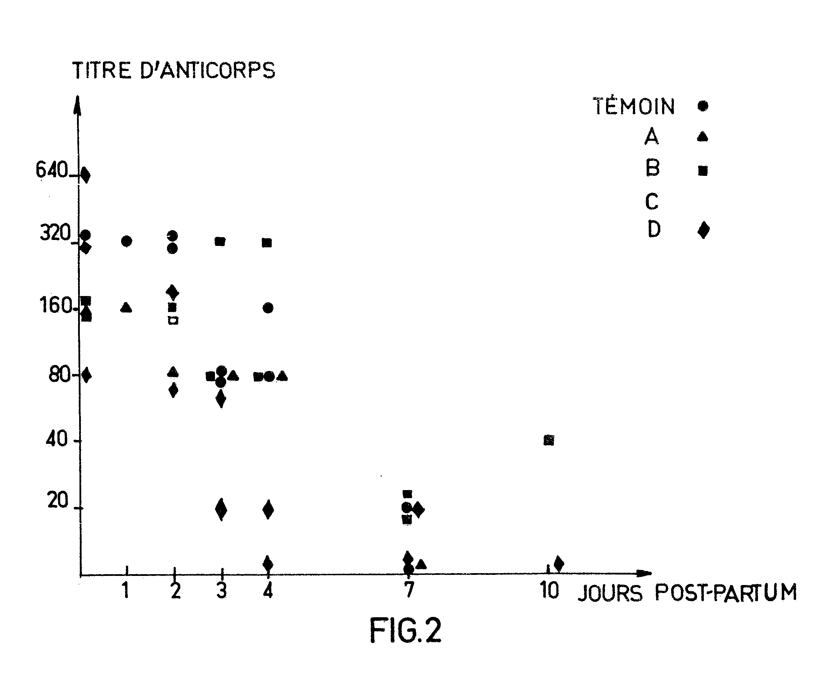

Chez 8 vaches appartenant à différents groupes, on a suivi la persistance des anticorps dans la sécrétion mammaire plusieurs jours après la mise bas. Les résultats ont été reportés sur la fig. 2 qui est un graphique où l'on a proté en ordonnées le titre d'anticorps Ac dans le lait et en abcisses le nombre de jours post-partum. Quel que soit le vaccin utilisé ou l'antigène, le taux d'anticorps est supérieur au 1/80e jour jusqu'à 2 jours. Passé ce délai, les anticorps disparaissent très rapidement (1/20e à 7 jours).

Ce résultat montre que si le veau boit correctement, il reçoit à chaque tétée une dose d'anticorps importante.

Il est protégé non seulement par l'absorption des anticorps et passage de ceux-ci dans le sang (protection contre la septicimie) mais aussi par apport continu d'anticorps au site même de l'éventuelle infection (tube digestif) pendant la période des 4 premiers jours de vie. Or, ces quatre premiers jours sont critiques car plus de 60% de la morbidité (diarrhée) est attribuable aux E.coli entéropathogènes.

### d) Efficacité des vaccins

Dans le tableau II, on a rassemblé les résultats obtenus parès infection expérimentale de veaux issus des vaches vaccinées et témoins.

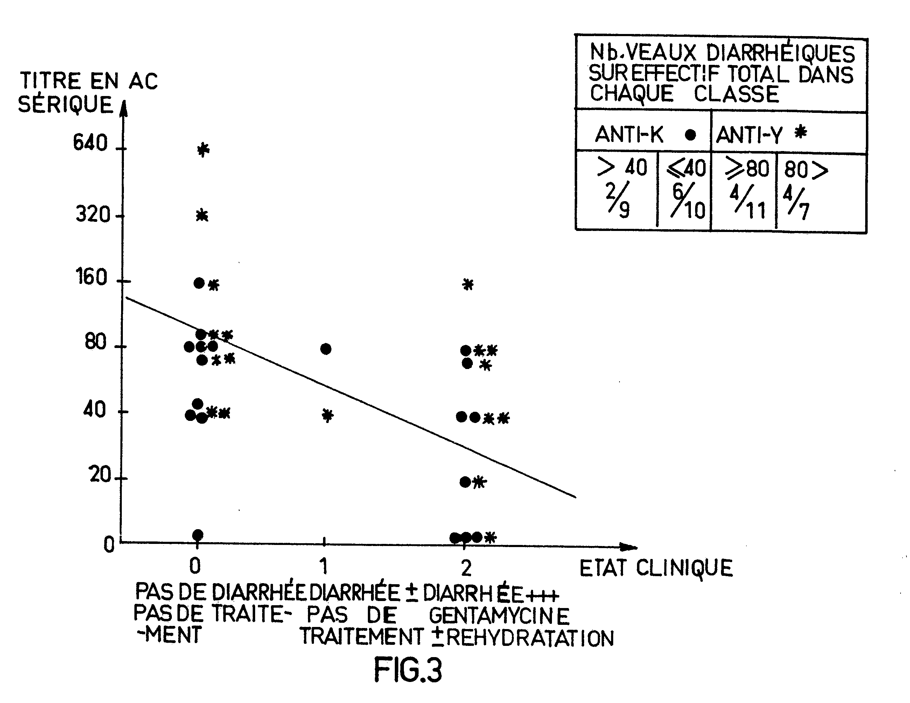

Ces résultats indiquent que les préparations A et D sont très efficaces (test de $\chi^2$ corrigé, haute signification). Par contre, la préparation B est inefficace malgré de bons titres en anticorps colostraux. Si l'on examine le titre en anticorps sérique chez les veaux à 48 h (figure 3) dans ce lot, on constate que chez 3 veaux ayant la diarrhée le titre en anti K99 était nul et chez le 4ème égal au 1/40ème. La figure 3 est un graphique illustrant l'état clinique des veaux et leur devenir en fonction du titre en anticorps sériques. Les légendes complètent la figure.

Pour l'ensemble des veaux, leur devenir est d'ailleurs dépendant du titre sérique en anticorps (figure 3). Si leur titre en anti K99 est supérieur au 1/40ème, ils ont peu de chance d'être malades. Par contre pour l'antigène Y, les titres en anticorps sont moins liés, ce qui est normal puisque la souche infectante est seulement K99+.

TABLEAU IV
Efficacité des vaccins testés

| Témoins | Nombre de veaux diarrhéiques |
|---------|------------------------------|
|         | 4/4                          |
| Lot A   | 0/5*)                        |
| Lot B   | 4/5**)                       |
| Lot C   |                              |
| Lot D   | 1/4*)                        |

*)   Test de $x^2$ corrigé hautement significatif.
**)  Test de $x^2$ corrigé non significatif.


### e) Conclusion

Dans des conditions expérimentales difficiles les résultats obtenus montrent l'intérêt des vaccins anticolibacillaires essayés. Les trois vaccins A, B et D ne comportent pas de risques de vaccination. Ces vaccins sont à injecter en une seule fois entre le 20ème et le 40ème jour avant la mise bas, donc dans la plupart des cas quand les vaches sont déjà à l'étable.

La protection obtenue chez les veaux est bonne puisqu'ils sont résistants à l'infection expérimentale par un E.coli pathogène, mais à condition que le veau boive correctement le colostrum.


### Exemple 6

On a préparé de la même manière qu'aux exemples 1 à 4 des vaccins A', B', C', D' correspondant respectivement aux vaccins A, B, C, D précédents en tuilisant la souche B41 et les trois souches de l'invention Y, 31A et 47A. On a ainsi obtenu des vaccins contenant les quatre antigènes respectifs. Des essais de protection des veaux nouveau-nés ont montré que les vaccins anticolibacillaires correspondants étaient efficaces. Le vaccin A' s'est avéré le plus intéressant.


### Exemple 7

Des échantillons de fèces de veaux atteints de diarrhée ont été collectés en zone Charolaise au cours de l'hiver 77—78. Après dissémination directe des matières fécales sur milieu Minca [Guinee, P.A.M. Veldkamp, J., Jansen, W.M. Improved Minca medium for the detection of K99 antigen calf enterotoxigenic strains of Escherichia coli. Infect. Immun. 15 (2): 676—678 (1977)] additionné de Polyvitex (Bio-Mérieux, Marcy l'Etoile-France, mélange de glucose et de facteurs de croissance) 5 souches par échantillon ont été repiquées sur milieu Minca gélosé, puis étudiées.

L'attachement des colibacilles à des villosités intestinales a été étudié selon la méthode in vitro de GIRARDEAU [Girardeau, J.P. A new in vitro technique for attachment to intestinal villi using enteropathogenic E.coli. Ann. Microbiol. Institut Pasteur (sous presse)], par observation directe au microscope.

L'antigène K99 a été recherché par agglutination sur lame avec un antisérum spécifique et la production de toxine TS selon la méthode du souriceau nouveau-né. [Dean, A.G. Ghing, Y., Williams, R.G. Harden, L.B. Test for Escherichia coli enterotoxin using infant mice: application in a study of diarrhea in Honolulu. J. Inf. Dis. 125; 407—411 (1972)].

Pour apprécier les potentialités pathogènes des E.coli ATT+, des veaux axéniques [Gouet, Ph., Riou, Y, Contrepois, M., Dubourguier, H.C. Efficacité d'une technique simple de production d'agneaux axéniques et de veaux oligoxéniques par décontamination à la naissance. Ann. Rech. Vet. 10 (1): 49—54 (1979)] ne recevant pas de colostrum ont été infectés à la naissance. L'évolution clinique a été observée et les veaux moribonds ont été autopsiés.

Pour mesurer l'effet protecteur des anticorps colostraux anti attachement, des vaches ont été vaccinées avec un antigène K99 partiellement purifié. L'antigène associé à l'adjuvant incomplet de FREUND a été administré par voie sous-cutanée. Le colostrum des vaches a été recueilli puis congelé. La protection conférée par le colostrum des vaches vaccinées a été appréciée par infection orale de veaux nouveau-nés ayant reçu 2 l du colostrum immun, comparativement à des veaux recevant un colostrum non immun.

Les résultats suivants ont été obtenus.

### Frequence des E.coli ATT+

### 1. Antigène K99

Six cents souches d'E.coli ont été isolées en provenance de 120 échantillons de fèces de veaux diarrhéiques. Trente pour cent des souches sont K99+E.coli. K99+ a été retrouvé dans les fèces de 40% des veaux âgés de un jour à un mois. Cette fréquence atteint 57% chez les veaux de moins de 4 jours. De plus, on a observé que parmi les 30% de souches K99+, seulement 14% expriment leur Ag K99 en l'absence de glucose dans le milieu de culture, alors que pour 16%, le glucose est indispensable (souches glucose dépendantes).

### 2. Autres facteurs d'attachement des colibacilles

Les propriétés d'attachement ont été recherchées sur un échantillonnage constitué de 47 souches K99+ et 53 souches K99⁻ représentatif de la totalité des veaux.

L'attachement sur villosités in vitro montre (tableau V) que 71% des souches de colibacilles s'attachent aux villosités intestinales, 30% grâce à l'Ag K99 et 41% grâce à des structures différentes.

L'étude par sérologie et par la microscopie électronique a montré que deux autres structures au moins sont responsables de l'attachement de colibacilles à l'intestin. L'une appelée F(Y) de 4 nm de diamètre, très longue comme l'Ag K99 et dont l'attachement aux villosités se révèle in vitro nettement plus intense que K99 seul. F(Y) est présente chez 18% des souches, soit seule (9%) soit en association avec une autre structure d'attachement appelée F(31A) (3%): plus courte et mesurant 5 nm de diamètre, elle est présente chez 8% des souches, soit seule (5%), soit en association avec F(Y).

Tableau V

Fréquence des souches bovines portant différentes structures d'attachement à l'épithélium intestinal K 99, F(Y), F(31 A)

| Souches en% | K99*) | | F(Y) | F(31A) | ATT | Entero-toxine TS | 0 nm |
|---|---|---|---|---|---|---|---|
| | 1 | 2 | | | | | |
| 14 | XXXXXXX | | | | + | + | 4 |
| 10 | | XXXXXXX | | | + | + | 4 |
| 6 | | XXXXXXX | XXXXXXX | | + | + | |
| 9 | | | XXXXXXX | | + | — | 4 |
| 3 | | | XXXXXXX | XXXXXXX | + | — | |
| 5 | | | | XXXXXXX | + | | 7 |
| 24 | | | | | + | — | |
| 29 | | | | | — | — | |

\*) K99 1 = glucose non dépendant
   K99 2 = glucose dépendant

### Mise en évidence chez le veau axénique du pouvoir pathogène d'E. coli portant la structure F (31A)

Dix veaux axéniques nouveau-nés privés de colostrum ont été inoculés avec 3 souches différentes (A, B, C) porteuses du facteur F(31A) (tableau VI) à raison de $10^{10}$ bactéries, par voie orale. Les souches A et B induisent une septicémie avec ou sans diarrhée. Elles provoquent des suffusions au niveau d'organes vitaux (coeur, rate). L'oedème péri-rénal est pratiquement toujours observé. L'atteinte nerveuse peut se traduire par des lésions histologiques cérébrales. Dans tous les cas, il y a broncho-pneumonie interstitielle plus ou moins prononcée pouvant aller jusqu'à l'emphysème. Avec la souche C, un veau sur trois est mort mais il n'a pas fait l'objet d'un examen post mortem.

TABLEAU VI

Résultats bactériologiques et cliniques de l'infection par les 3 souches A, B, C d'E. coli F(31A)

| | Nombre E. coli/g | | | Veaux morts |
|---|---|---|---|---|
| | Liquide céphalorachidien | sang du coeur | urine | |
| E. coli A | $2 \times 10^6$ | $10^8$ | $10^7$ | 4/4 |
| E. coli B | $10^6$ | $10^5$ | $10^3$ | 2/3 |
| E. coli C | non fait | non fait | non fait | 1/3 |

Effets protecteurs d'une vaccination anti K99

La vaccination avec un antigène K99 obtenu à partir de la souche B41 (0101 : K99 : H⁻) par précipitation à pH 4 du surnageant du mélange tampon-bactéries après chauffage et agitation 20 mn à 60°C a permis d'obtenir un colostrum contenant des anticorps contre l'Ag K99 (tableau VII). Des anticorps anti 0 sont aussi apparus. Les titres en agglutinines anti 0 sont multipliés par 8 dans le colostrum des vaches vaccinées comparativement aux témoins et les agglutines anti K99 totalement absentes chez les témoins, atteignent en moyenne 200 dans le colostrum des animaux vaccinés.

TABLEAU VII

Titres en anticorps dans les colostrum

| | | 1 | 2 | 3 | 4 | 5 | 6 | 7 | |
|---|---|---|---|---|---|---|---|---|---|
| Vaches | $A_1$ | 160 | 80 | 80 | 80 | 20 | 20 | 0 | $m \simeq 40$ |
| témoins | $B_1$ | 0 | 0 | 0 | 0 | 0 | 0 | 0 | $m \simeq 0$ |
| Vaches | $A_1$ | 320 | 320 | 320 | 160 | | | | $m \simeq 320$ |
| vaccinées | $B_1$ | 320 | 320 | 160 | 80 | | | | $m \simeq 200$ |

Les titres sont l'inverse de la dilution la plus élevée donnant une agglutination visible en tubes.

$A_1$ = souche 0101 : K99 : H⁻ incubée à 18°C (Ac anti Ag somatiques)
$B_1$ = souche 09 : K99 : H⁻ incubée à 37°C (Ac anti K99)

Dans le cas d'une infection orale par la souche B41 les veaux recevant le colostrum immun sont protégés presqu'à 100% (tableau VIII). Dans le cas d'une infection par une souche hétérologue K99+ (09 : K30,K99 : H⁻) la protection est moins bonne puisque 3 animaux seulement sur 6 ont été totalement protégés et que 2 sont même morts.

TABLEAU VIII

Protection conférée par un colostrum anti K99 chez des veaux infectés expérimentalement par E. coli K99⁺, TS⁺

| Souche infectante | Nombre de veaux | Diarrhée | Déshydra- tation | Mort |
|---|---|---|---|---|
| 0101 : K99 : H⁻ | témoins: 4 | 4 | 3 | 2 |
| | vaccinés: 6 | 1 | 0 | 0 |
| 09 : K30, K99 : H⁻ | témoins: 7 | 7 | 7 | 6 |
| | vaccinés: 6 | 3 | 2 | 2 |

Le facteur F(Y) d'une morphologie proche de K99 peut coexister avec K99 ou F(31A). Exception faite des souches F(Y) qui sont aussi K99⁺, la toxine TS n'a pu être décélée chez les souches F(Y). On a également observé in vitro que l'attachement des souches K99⁺ F(Y) est beaucoup plus intense que celui des souches K99, ce qui peut aggraver la pathogénicité de ce type d'E.coli.

Les trois souches F(31A) qui ont servi à infecter des veaux axéniques ont des potentialités pathogènes certaines, puisqu'elles produisent une septicémie et des lésions entraînant la mort des animaux en moins de 48 heures. Il n'y a pas d'analogie avec les souches invasies d'E.coli possédant un plasmide Vir.

Ces résultats confirment que les facteurs d'attachement d'E.coli entéropathogènes et septicémiques ont une très grande importance.

## Revendications

1. Vaccin anticolibacillaire animal, caractérisé en ce qu'il contient l'antigène K99 et au moins un antigène provenant des souches d'E.coli choisies parmi les souches Y+, 31A+ et 47A+.

2. Vaccin selon la revendication 1, caractérisé en ce qu'il contient les antigènes K99 et Y.

3. Vaccin selon la revendication 1, caractérisé en ce qu'il contient les antigènes K99 et 31A.

4. Vaccin selon la revendication 1, caractérisé en ce qu'il contient les antigènes K99, Y et 31A.

5. Vaccin selon l'une quelconque des revendications 2 à 4, caractérisé en ce qu'il contient en outre l'antigène 47A.

6. Procédé pour l'obtention d'un vaccin anticolibacillaire animal contenant l'antigène K99 et au moins un antigène provenant des souches Y+, 31A+ et 47A+, caractérisé en ce qu'on cultive séparément les souches d'E.coli portant lesdits antigènes, on récolte les bactéries ainsi respectivement cultivées, on mélange les cultures de bactéries respectives, on inactive le mélange de bactéries et on l'additionne éventuellement d'un adjuvant, ce qui conduit à la préparation vaccinale désirée.

7. Procédé selon la revendication 6, dans lequel le milieu de culture des souches d'E.coli, est un milieu aqueux gélosé tamponné contenant entre autres, des acides aminés, du glucose et des éléments minéraux, caractérisé en ce que ledit milieu de culture contient au plus 0,3 g/l de L-alanine.

8. Procédé selon l'une des revendications 6 ou 7, caractérisé en ce que la souche portant l'antigène K99 est la souche B41.

9. Procédé selon la revendication 7, caractérisé en ce que le milieu de culture contient en outre de l'acide aspartique.

10. Procédé selon l'une quelconque des revendications 6 à 9, caractérisé en ce que le milieu de culture présente la composition suivante (en g/l):

| | |
|---|---|
| — milieu gélosé | 10 à 20 g d'agar |
| — milieu tamponné | à pH 7,5 (phosphates) |
| — acides aminés (peptone) | 1 à 5 g |
| — glucose | 1 à 5 g |
| — acid aspartique | 0 à 4 g |
| — désoxycholate de Na | 0 à 2 g |
| — extraits de levures | 0 à 1 g |
| — éléments minéraux (MgSO₄, McCl₂, FeCl₂, CaCl₂) | de 0,01 à 0,0001 g |

11. Procédé selon l'une quelconque des revendications 6 à 10, caractérisé en ce qu'on inactive les bactéries par le formol ou de préférence par irradiation à l'aide de rayons gamma.

14

12. Procédé selon l'une quelconque des revendications 6 à 11, caractérisé en ce qu'à la préparation vaccinale contenant les bactéries inactivées, on ajoute, à titre d'adjuvant, de l'alumine colloidale à raison de 1 à 3 mg/ml par rapport à la solution finale.

13. Procédé selon la revendication 12, caractérisé en ce qu'on ajoute complémentairement de la saponine, à raison de 0,5 à 3 mg pour un volume total de solution finale de 2 à 5 ml.

14. Procédé selon l'une quelconque des revendications 6 à 13, caractérisé en ce qu'on utilise les bactéries E.coli respectives pour fournir une préparation vaccinale contenant les antigènes K99 et Y, et/ou 31A et/ou 47A, en proportions sensiblement égales.

15. Procédé selon la revendication 14, caractérisé en ce que la préparation vaccinale contient $10^9$ à $10^{10}$ bactéries par ml.

16. Préparation vaccinale obtenue par le procédé selon l'une quelconque des revendications 6 à 15.

17. Préparation vaccinale contenant $10^9$ à $10^{10}$ bactéries E.coli inactivées portant les antigènes K99 et Y, et/ou 31A et/ou 47A, ceux-ci étant présents en proportions sensiblement égales dans la préparation.

18. Préparation vaccinale selon la revendication 15, caractérisée en ce qu'elle se présente sous une forme appropriée pour injection sous-cutanée.

19. Souche de E.coli caractérisée en ce qu'elle comporte l'antigène de surface F(31A) reconnaissable en séroagglutination à l'aide d'un antisérum spécifique de cet antigène, préparé à partir de la souche type (no. de collection I-105 à l'Institut Pasteur), ledit antisérum ne reconnaissant ni l'antigène K99 (souche de référence B41) ni l'antigène Y (no. de collection I-104) ni l'antigène 47A (no. de collection I-106).

20. Souche de E.coli caractérisée en ce qu'elle comporte l'antigène de surface F(Y) reconnaissable en séroagglutination à l'aide d'un antisérum spécifique de cet antigène, préparé à partir de la souche type (no. de collection I-104), ledit antisérum ne reconnaissant ni l'antigène K99 (souche de référence B41) ni l'antigène 31A (no. de collection I-105) ni l'antigène 47A (no. de collection I-106).

21. Souche de E.coli caractérisé en ce qu'elle comporte l'antigène de surface F(47A) reconnaissable en séroagglutination à l'aide d'un antisérum spécifique de cet antigène, préparé à partir de la souche type (no. de collection I-106), ledit antisérum ne reconnaissant ni l'antigène K99 (souche de référence B41) ni l'antigène 31A (no. de collection I-105) ni l'antigène Y (no. de collection I-104).

## Patentansprüche

1. Tierimpfstoff gegen den Kolibazillus, dadurch gekennzeichnet, daß er das Antigen K99 und wenigstens ein Antigen enthält, das von Stämmen von E. coli stammt, die aus den Stämmen Y+, 31A+ und 47A+ ausgewählt sind.

2. Impfstoff nach Anspruch 1, dadurch gekennzeichnet, daß er die Antigene K99 und Y enthält.

3. Impfstoff nach Anspruch 1, dadurch gekennzeichnet, daß er die Antigene K99 und 31A enthält.

4. Impfstoff nach Anspruch 1, dadurch gekennzeichnet, daß er die Antigene K99, Y und 31A enthält.

5. Impfstoff nach irgendeinem der Ansprüche 2 bis 4, dadurch gekennzeichnet, daß er außerdem das Antigen 47A enthält.

6. Verfahren zur Herstellung eines Tierimpfstoffes gegen den Kolibazillus, der das Antigen K99 und wenigstens ein von den Stämmen Y+, 31A+ und 47A+ stammendes Antigen enthält, dadurch gekennzeichnet, daß man getrennt die Stämme von E. coli, die die genannten Antigene enthalten, kultiviert, die in dieser Weise jeweils kultivierten Bakterien gewinnt, die jeweiligen Bakterienkulturen mischt, das Bakteriengemisch deaktiviert und es gegebenenfalls mit einem Adjuvans versetzt, was zum gewünschten Impfstoffpräparat führt.

7. Verfahren nach Anspruch 6, worin das Kulturmedium der Stämme von E. coli ein gepuffertes wäßriges Gelose-Medium ist, das u. a. Aminosäuren, Glucose und Mineralstoffe enthält, dadurch gekennzeichnet, daß das Kulturmedium höchstens 0,3 g/l L-Alanin enthält.

8. Verfahren nach einem der Ansprüche 6 oder 7, dadurch gekennzeichnet, daß der das Antigen K99 enthaltende Stamm der Stamm B41 ist.

9. Verfahren nach Anspruch 7, dadurch gekennzeichnet, daß das Kultumedium außerdem Asparaginsäure enthält.

10. Verfahren nach irgendeinem der Ansprüche 6 bis 9, dadurch gekennzeichnet, daß das Kulturmedium die folgende Zusammensetzung (in g/l) hat:

| Gelosemedium | 10 bis 20 g Agar |
|---|---|
| auf pH 7,5 gepuffertes Medium (Phosphate) | |
| Aminosäuren (Pepton) | 1 bis 5 g |
| Glukose | 1 bis 5 g |
| Asparaginsäure | 0 bis 4 g |
| Na-Desoxycholat | 0 bis 2 g |
| Hefeextrakte | 0 bis 1 g |
| Mineralstoffe ($MgSO_4$, $MnCl_2$, $FeCl_2$, $CaCl_2$) | 0,01 bis 0,0001 g |

11. Verfahren nach irgendeinem der Ansprüche 6 bis 10, dadurch gekennzeichnet, daß man die Bakterien mit Formol oder vorzugsweise durch Bestrahlung mit Gammastrahlen inaktiviert.

12. Verfahren nach irgendeinem der Ansprüche 6 bis 11, dadurch gekennzeichnet, daß man zum Impfstoffpräparat, das die inaktivierten Bakterien enthält, als Adjuvans kolloidales Aluminiumoxid in einer Menge von 1 bis 3 mg/ml, bezogen auf die endgültige Lösung, gibt.

13. Verfahren nach Anspruch 12, dadurch gekennzeichnet, daß man als Ergänzung Saponin in einer Menge von 0,5 bis 3 mg pro Gesamtvolumen von 2 bis 5 ml der endgültigen Lösung gibt.

14. Verfahren nach irgendeinem der Ansprüche 6 bis 13, dadurch gekennzeichnet, daß man die jeweiligen Bakterien E. coli verwendet, um ein Impfstoffpräparat zu liefern, das die Antigene K99 und Y und/oder 31A und/oder 47A in im wesentlichen gleichen Anteilen enthält.

15. Verfahren nach Anspruch 14, dadurch gekennzeichnet, daß das Impfstoffpräparat $10^9$ bis $10^{10}$ Bakterien pro ml enthält.

16. Impfstoffpräparat, erhalten nach dem Verfahren gemäß irgendeinem der Ansprüche 6 bis 15.

17. Impfstoffpräparat, enthaltend $10^9$ bis $10^{10}$ inaktivierte Bakterien E. coli, die die im wesentlichen gleichen Anteile im Präparat enthaltenen Antigene K99 und Y und/oder 31A und/oder 47A enthalten.

18. Impfstoffpräparat nach Anspruch 15, dadurch gekennzeichnet, daß es in einer für die subkutane Injektion geeigneten Form vorliegt.

19. Stamm von E. coli, dadurch gekennzeichnet, daß er das Oberflächenantigen F(31A) enthält, das in der Seroagglutination mit Hilfe eines für dieses Antigen spezifischen Antiserums erkennbar ist, hergestellt aus dem Typstamm (Hinterlegungsnummer I-105 beim Pasteur-Institut), wobei das genannte Antiserum weder das Antigen K99 (Bezugsstamm B41) noch das Antigen Y (Hinterlegungsnummer I-104) noch das Antigen 47A (Hinterlegungsnummer I-106) erkennt.

20. Stamm von E. coli, dadurch gekennzeichnet, daß er das Oberflächenantigen F(Y) enthält, das in der Seroagglutination mit Hilfe eines für dieses Antigen spezifischen Antiserums erkennbar ist, hergestellt aus dem Typstamm (Hinterlegungsnummer I-104), wobei das genannte Antiserum weder das Antigen K99 (Bezugsstamm B41) noch das Antigen 31A (Hinterlegungsnummer I-105) noch das Antigen 47A (Hinterlegungsnummer I-106) erkennt.

21. Stamm von E. coli, dadurch gekennzeichnet, daß er das Oberflächenantigen F(47A) enthält, das in der Seroagglutination mit Hilfe eines für dieses Antigen spezifischen Antiserums erkennbar ist, hergestellt aus dem Typstamm (Hinterlegungsnummer I-106), wobei das genannte Antiserum weder das Antigen K99 (Bezugsstamm B41) noch das Antigen 31A (Hinterlegungsnummer I-105) noch das Antigen Y (Hinterlegungsnummer I-104) erkennt.

## Claims

1. Animal anti-colibacillary vaccine, characterized in that it contains K99 antigen and at least one antigen originating from strains of E.coli selected among the $Y^+$, $31A^+$ and $47A^+$ strains.

2. Vaccine according to claim 1, characterized in that it contains the K99 and Y antigens.

3. Vaccine according to claim 1, characterized in that it contains the K99 and 31A antigens.

4. Vaccine according to claim 1, characterized in that it contains the K99, Y and 31A antigens.

5. Vaccine according to anyone of claims 2 to 4, characterized in that it further contains the 47A antigen.

6. Process for obtaining an animal anti-colibacillary vaccine containing K99 antigen and at least one antigen originating from $Y^+$, $31A^+$ and $47A^+$ strains, characterized in that the strains of E.coli, bearing the antigens considered are separately cultivated, the thus respectively cultivated bacteria are collected, the respective bacterial cultures are mixed, the bacterial mixture is inactivated and an additive is added, optionally, which leads to the desired vaccinal preparation.

7. Process according to claim 6, in which the culture medium of the strains of E.coli is a buffered, aqueous agar medium containing, inter alia, amino acids, glucose and mineral salts, characterized in that said culture medium contains at most 0.3 g/l of L-alanine.

8. Process according to anyone of claims 6 or 7, characterized in that the strain bearing the K99 antigen is B41 strain.

9. Process according to claim 7, characterized in that the culture medium further contains aspartic acid.

10. Process according to anyone of claims 6 to 9, characterized in that the culture medium has the following composition (in g/l):

| | |
|---|---|
| agar medium | 10 to 20 g of agar |
| medium buffered | to pH 7,5 (phosphates) |
| amino acids (peptone) | 1 to 5 g |
| glucose | 1 to 5 g |
| aspartic acid | 0 to 4 g |
| Na deoxicholate | 0 to 2 g |
| yeast extracts | 0 to 1 g |
| mineral salts ($MgSO_4$, $MnCl_2$, $FeCl_2$, $CaCl_2$) | from 0.01 to 0.0001 g |

11. Process according to anyone of claims 6 to 10, characterized in that the bacteria are inactivated by formol or preferably by irradiation with gamma rays.

12. Process according to anyone of claims 6 to 11, characterized in that colloidal alumina as an additive is added to the vaccinal preparation containing inactivated bacteria in the proportion of 1 to 3 mg/ml with respect to the final solution.

13. Process according to claim 12, characterized in that saponin is supplementarily added in the proportion of 0.5 to 3 mg for a total volume of the final solution of 2 to 5 ml.

14. Process according to anyone of claims 6 to 13, characterized in that the respective E.coli bacterial are used to furnish a vaccinal preparation containing antigens K99 and Y, and/or 31A and/or 47A in substantially equal proportions.

15. Process according to claim 14, characterized in that the vaccinal preparation contains $10^9$ to $10^{10}$ bacteria per ml.

16. Vaccinal preparation obtained by the process according to anyone of claims 6 to 15.

17. Vaccinal preparation containing $10^9$ to $10^{10}$ inactivated E.coli bacteria bearing the antigens K99 and Y, and/or 31A and/or 47A, these being present in substantially equal proportions in the preparation.

18. Vaccinal preparation according to claim 17, characterized in that it is in a form suitable for sub-cutaneous injection.

19. Strain of E.coli, characterized in that it comprises the F(31A) surface antigen recognizable by seroagglutination with a specific antiserum of this antigen, prepared from the standard strain (collection n° I-105 at the Institut Pasteur), said antiserum recognizing neither the K99 antigen (reference strain B41), nor the Y antigen (collection n° I-104), nor the 47A antigen (collection n° I-106).

20. Strain of E.coli, characterized in that it comprises the F(Y) surface antigen recognizable by seroagglutination with a specific antiserum of this antigen, prepared from the standard strain (collection n° I-104), said antiserum recognizing neither the K99 antigen (reference strain B41), nor the 31A antigen (collection n° I-105) nor the 47A antigen (collection n° I-106).

21. Strain of E.coli, characterized in that it comprises the F(47A) surface antigen recognizable by seroagglutination with a specific antiserum of this antigen, prepared from the standard strain (collection n° I-106), said antiserum recognizing neither the K99 antigen (reference strain B41) nor the 31A antigen (collection n° I-105) nor the Y antigen (collection n° I-104).

FIG.1

FIG.2

FIG.3